# EUROPEAN PATENT SPECIFICATION

(11) **EP 1 373 273 B1**
(45) Date of publication and mention of the grant of the patent: **05.10.2005**
(21) Application number: 02726608.9
(22) Date of filing: 12.03.2002
(51) Int. Cl.: C07D 487/12, A61K 31/53

(54) **PROCESS FOR PREPARING MAYTANSINOL**
VERFAHREN ZUR HERSTELLUNG VON MAYTANSINOL
PROCEDES RELATIFS A L'ELABORATION DE MAYTANSINOL

(30) Priority: 16.03.2001 US 276792 P
(43) Date of publication of application: 02.01.2004
(73) Proprietor: SMITHKLINE BEECHAM CORPORATION, Philadelphia, Pennsylvania 19101 (US)
(72) Inventor: TERFLOTH, Gerald, J., King of Prussia, PA 19406 (US)
(74) Representative: Connell, Anthony Christopher
(86) International application number: PCT/US2002/007424
(87) International publication number: WO 2002/074775

(56) References cited:
- WO-A-02/16368
- US-A- 4 162 940
- US-A- 5 416 064
- KUPCHAN S M ET AL: "Structural Reqirements for Antileukemic Activity among the Naturally Occurring and Semisynthetic Maytansinoids" JOURNAL OF MEDICINAL CHEMISTRY, AMERICAN CHEMICAL SOCIETY. WASHINGTON, US, vol. 21, no. 1, 1978, pages 31-37, XP002278688 ISSN: 0022-2623
- REICH E ET AL: "Normal- and bonded-phase liquid chromatography with photodiode array detection of maytansinoids" JOURNAL OF CHROMATOGRAPHY A, ELSEVIER SCIENCE, NL, vol. 763, no. 1-2, 28 February 1997 (1997-02-28), pages 213-219, XP004116749 ISSN: 0021-9673
- CHARI R V J ET AL: "IMMUNOCONJUGATES CONTAINING NOVEL MAYTANSINOIDS: PORMISING ANTICANCER DRUGS" CANCER RESEARCH, AMERICAN ASSOCIATION FOR CANCER RESEARCH, BALTIMORE, MD, US, vol. 52, no. 1, 1992, pages 127-131, XP000453560 ISSN: 0008-5472

## Description

### Field of the Invention

This invention relates to processes for preparing maytansinol using high-performance liquid chromatography.

### Background of the Invention

Highly cytotoxic maytansinoid drugs and their therapeutic use have been described in U.S. Patent No. 5,208,020. These drugs can be prepared from maytansinol derivatives which are in turn prepared from ansamitocin precursors.

A number of related ansamitocins can be produced under defined culture conditions from *Actinosynnema spp.* such as *Actinosynnema pretosium.* Processes for ansamitocin production from *Actinosynnema spp.* have been described in U.S. Patent Nos. 4,162,940; 4,228,239; 4,356,265; and 4,450,234.

Maytansinol can be prepared by reductive cleavage of ansamitocin C-3 esters. Following reduction, it is necessary to purify maytansinol from reaction mixtures for further derivitazation to produce compounds such as the N-methyl-L-alanine derivative.

General methods for the purification of maytansinol from reaction mixtures can include extraction, distillation, crystallization or open-column chromatography. In general, open-column chromatography yields the purest material but is difficult to scale from an investigational lab-scale to a production scale. Problems that are encountered include irreproducible sample introduction, variability in column-bed efficiency, cycle time variability, operator-dependent variability in product quality and yield and a low cost-benefit for the entire process. Thus, a need exists for a reproducible and scalable preparative chromatography method for the purification of maytansinol.

### Summary of the Invention

One aspect of the invention is a process for preparing maytansinol from a mixture containing unreduced and over-reduced maytansinoids by separating the maytansinol by normal-phase high performance liquid chromatography on an angular particle porous amorphous silica gel stationary phase.

### Brief Description of the Drawing

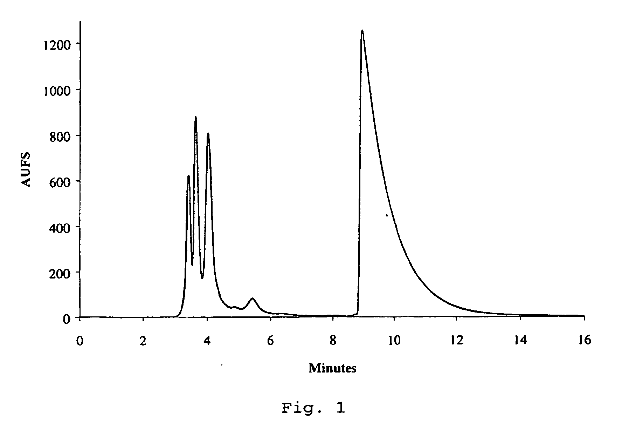

Fig. 1 shows a chomatographic separation of maytansinol on a silica gel stationary phase.

### Detailed Description of the Invention

All publications, including but not limited to patents and patent applications, cited in this specification are herein incorporated by reference as though fully set forth.

By the term "high performance liquid chromatography (HPLC)" as used herein and in the claims is meant a process in which a chemical mixture carried by a liquid mobile phase is separated into components as a result of differential distribution of the components as they flow around, over or countercurrent to a stationary solid phase. The mobile phase is composed of 50% dichloromethane : 39,3% ethyl acetate : 10,7% 2-propanol having a flowrate of 0.001 to 50 cm/s under a pressure of 1 to 400 bar.

Processes are provided for preparing maytansinol (II) from a mixture containing unreduced and over-reduced maytansinoids by separating the maytansinol by normal-phase HPLC on an angular particle porous amorphous silica gel stationary phase.

As shown in Scheme I, maytansinoid esters having a C-3 acyl side chain such as maytanacine, also known as ansamitocin P-1 (**Ia**), P-2 (**Ib**), P-3 (**Ic**), P-3' (**Id**), P-4 (**Ie**) and P-4' (**If**) can undergo reductive hydrolyis to produce maytansinol (**II**), also known as form P-0.

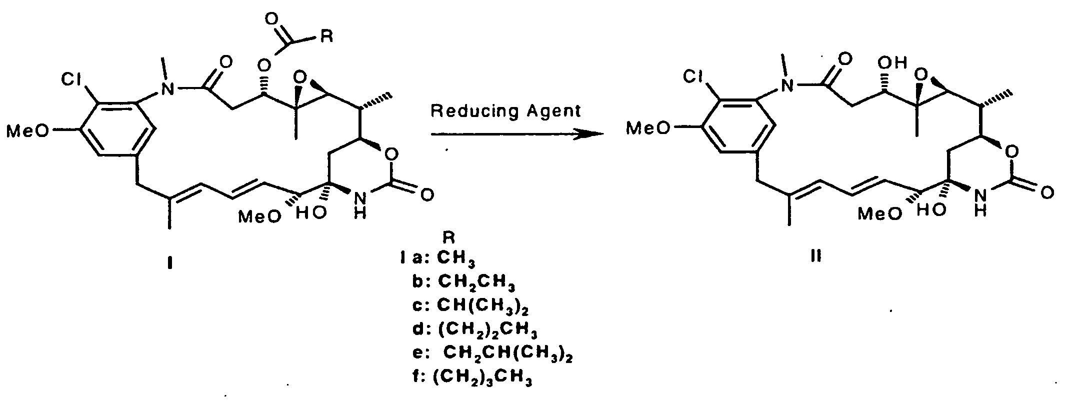

The reductive hydrolysis reaction can be carried out as follows. A maytansinoid C-3 ester (**Ia-f**) is dissolved in an anhydrous solvent, then placed under an inert gas atmosphere and cooled. Preferably, the maytansinoid C-3 ester is maytanacine (**Ia**) or ansamitocin P-3 (**Ic**). Maytanacine (**Ia**) can be obtained as described by Kupchan et al. in *J. Org. Chem. 42,* 2349-2357 (1977). Processes for the preparation of ansamitocins P-2, P-3, P-4 and P-4' are described in Hatano *et al*., *Agric. Biol. Chem 48,* 1721-1729 (1984). P-3' can be obtained as described in Tanida *et al., J. Antibiot. (Tokyo) 34,* 489-495 (1981).

Preferably, the anhydrous solvent is tetrahydrofuran (THF), 2-methoxyethyl ether, dioxane or diethyl ether, although other solvents can be used. More preferably, the anhydrous solvent is tetrahydrofuran. Preferably, 20 to 30 mL of the anhydrous solvent is used per gram of maytansinoid ester, more preferably 25 mL/g. Preferably, the inert gas is argon, nitrogen or helium, although other gases can be used. More preferably, the inert gas is argon. Preferably, the solution is maintained at between about 0 to -80°C, more preferably between about -30 to -50°C, most preferably between about -35 to -45°C, in a dry ice-acetone bath.

A reducing agent is also cooled, and then transferred into the chilled solution of the maytansinoid C-3 ester. The reaction is maintained under an inert gas atmosphere, at a low temperature, and stirred. Preferably, the reducing agent is lithium trimethoxyaluminum hydride (LiAl(OMe)₃H), lithium triethoxyaluminum hydride (LiAl(OEt)₃H), lithium tripropoxyaluminum hydride (LiAl(OPr)₃H), sodium trimethoxyaluminum hydride (NaAl(OMe)₃H), sodium triethoxyaluminum hydride (NaAl(OEt)₃H) or sodium tripropoxyaluminum hydride (NaAl(OPr)₃H). More preferably, the reducing agent is lithium trimethoxyaluminum hydride (LiAl(OMe)₃H). Preferably, the reducing agent is cooled to about -30 to -40°C in a dry ice-acetone bath. Preferaby, the cooled reducing agent is transferred into the chilled solution of the maytansinoid C-3 ester via a cannula. Preferably, the inert gas is argon, nitrogen or helium, although other gases can be used. More preferably, the inert gas is argon.

Preferably, the reducing agent is used in a concentration of from about 5 to 100 equivalents per mole of the maytansinoid C-3 ester, more preferably from about 7.5 to 30 equivalents per mole, most preferably from about 10 to 20 equivalents per mole. One of ordinary skill in the art will understand that use of reducing agent in amounts greater than about 100 equivalents per mole of the maytansinoid C-3 ester can result in undesired side products. Preferably, the reducing agent is added to the chilled solution of maytansinoid C-3 ester over a time period ranging from about 5 to 40 minutes, more preferably from about 7 to 20 minutes, most preferably from about 8 to 12 minutes. Preferably, the reaction is maintained under an inert gas atmosphere at a temperature range of from about -25°C to -80°C, more preferably from about -27°C to -45°C, most preferably from about -30°C to -35°C. Preferably, the reaction is stirred between about 30 min and three hours, more preferably for about three hours.

One of ordinary skill in the art will understand that the amount of reducing agent used, the temperature maintained during the reaction, the length of the time period over which the reducing agent is added and the reaction time are each dependent on the other. For example, the lower the amount of the reducing agent, the longer the reaction time. Similarly, the lower the temperature, the larger the excess of reducing agent required and the longer the time required for completion of the reaction. Moreover, the slower the rate the reducing agent is added, the longer reaction time required for completion of the reaction.

The reaction is quenched, extracted, dried and filtered. The solvent is evaporated under reduced pressure to yield crude maytansinol. Preferably, the reaction is quenched by the addition of saturated sodium chloride solution, water or ammonium chloride solution, more preferably by the addition of saturated sodium chloride solution. Preferably, the reaction is quenched using between about 20 to 40 mL of the solution per gram of maytansinoid ester used. Preferably, the reaction is extracted with ethyl acetate, dichloromethane, toluene, chloroform or ether, more preferably with ethyl acetate. Preferably, the reaction is extracted at the rate of between about 4 x 80 to 4 x 200 mL/g maytansinoid ester used. Preferably, the combined ethyl acetate extracts are dried over sodium sulfate or magnesium sulfate, more preferably sodium sulfate, and filtered.

In one embodiment of the invention, the crude maytansinol is purified from unreduced and over-reduced maytansinoids by HPLC on a silica stationary phase eluted with 50% dichloromethane : 39,3% ethyl acetate : 10,7% 2-propanol.

In one preferred embodiment, the silica stationary phase is porous amorphous silica gel having a median pore diameter of 50-70 Å, a pore volume of 0.8-1.2 mL/g, a surface area of 500-600 m²/g, a packed density of 0.5 g/mL, <10% loss on drying for 20 min at 205°C and a 5% aqueous slurry pH of 4.0-5.5. Median pore diameter and pore volume are determined by N2 sorbtion. Surface area is determined using the Brunauer, Emmett and Teller (BET) method. Sodium, aluminum, iron and calcium, as determined by atomic absorption or inductively coupled plasma (ICP) of HF digests is <60 ppm, <100 ppm, <80 ppm and <80 ppm, respectively. Sulfate and chloride, as determined by ion chromatography of aqueous extracts are both individually <25 ppm. The pore diameter of the silica stationary phase can be 60, 100 or 200 Å. Most preferably, the pore diameter is 60 Å. The particle size of the silica stationary phase can be 5, 10, 20, 40, 80 or 150 µm. Preferably, the particle size is 20 or 40 µm. The preferred silica stationary phases can be prepared as described in U.S. Pat. Nos. 4,131,542 and 5,108,595 or are available as IMPAQ® silica gels from SiliCycle, Inc., Québec, Qc, Canada. The mobile phase is 50% dichloromethane: 39.3% ethyl acetate: 10.7% 2-propanol.

In another preferred embodiment, the silica stationary phase is silica gel having a median pore diameter of 60 Å, a pore volume of about 0.9 mL/g, a surface area of about 700 m²/g, a bulk density of about 0.4 g/mL, a 15% aqueous slurry pH of 6.5-7.5, an iron content of <0.02%, and chloride content of <0.02%. Silica gel meeting these specifications is available as Merck LICHROSPHER® Si 60 from E. Merck, Darmstadt, Germany.
Isoeluotropic eluents are described by L.R. Snyder and J.J. Kirkland in *Introduction to Modern Liquid Chromatography,* Wiley and Sons, New York, pp. 255-271 (1974).

In either embodiment, the chromatography is conducted within a temperature range of about -80°C to about 200°C, a linear flow rate of about 0.08 to about 1.6 cm/s under a pressure of about 5 to about 100 bar. Preferably, the temperature is about 20°C to about 25°C. Particularly preferred is ambient temperature.

It if further understood by those skilled in the art that the processes of the invention can be run in batch or continuous chromatography modes. An exemplary continuous mode is simulated moving bed chromatography. See Charton *et al*., *J. Chromatogr. 702,* 97-112 (1995).

The process of the invention can be used to make cell-binding agent/maytansinoid complexes which are useful as tumor-activated pro-drugs. Maytansinol produced by the process of the invention can be used as described in U.S. Patent No. 5,208,020 to produce N-methyl-L-alanine containing maytansinoid derivatives. These derivatives are then conjugated to cell-binding agents, preferably antibodies, via various linkers such as a disulfide link.

An exemplary cell-binding agent/maytansinoid complex can be prepared by a process comprising the following steps:
(1) esterifying maytansinol with N-methyl-L-alanine derivatives to form a disulfide-containing maytansinoid ester;
(2) reducing the disulfide-containing maytansinoid ester prepared by step (1) to a thiol-containing maytansinoid;
(3) introducing dithiopyridyl groups into a cell-binding agent; and
(4) linking the thiol-containing maytansinoid produced by step (2) to the dithiopyridyl cell-binding agent of step (3) by a disulfide link.

The present invention will now be described with reference to the following specific, non-limiting examples.

### Example 1

### Purification of Maytansinol by Large-Scale Preparative HPLC

Chromatography was carried out using a Varex preparative HPLC system with pump, feed pump and variable wavelength UV detector. A 101.6 mm inside diameter x 250 mm stainless steel column was packed with approximately 1 kg of IMPAQ® silica gel, an porous amorphous silica gel stationary phase having a pore diameter of 50-70 Å, a pore volume of 0.8-1.2 mL/g, a surface area of 500-600 m²/g, a packed density of 0.5 g/mL, <10% loss on drying for 20 min at 205°C, a 5% aqueous slurry pH of 4.0-5.5, sodium content of <60 ppm, aluminum content of <100 ppm, iron content of <80 ppm, calcium content of <80 ppm, sulfate content of <25 ppm and chloride content of <25 ppm and a particle size of 10 µm. This silica gel can be obtained from SiliCycle, Inc. as IMPAQ® 60A, 10 µm, under product number B1007B. A mobile phase of methylene chloride : ethyl acetate : 2-propanol (50 : 39.3 : 10.7) at a flow rate of 500 mL/min (0.145 cm/s) was used. Detection was at 280 nm. The column was equilibrated for at least 10 minutes before each run.

10.8 g of impure maytansinol prepared by lithium trimethoxyaluminum hydride reduction of ansamitocin P-3 was dissolved in 217 mL of mobile phase to give a concentration of 49.8 mg/mL. This sample solution was recirculated for 10 minutes through a 10 mm ID x 40 mm column (packed with the same silica as the preparative column) prior to pumping onto the preparative column. The analytical injection of the sample solution had a retention time of ≅ 13 minutes. A 3 gram run (Run 1) and two ≅ 4 gram runs (Runs 2 and 3) were made. An 80 ml injection was made for a 4 gram run.

A chromatogram obtained on a 4.6 mm inside diameter x 250 mm column is shown in Fig. 1. The compound of interest is well separated from earlier and later eluting impurities. A small fraction (Fr1, 125 mL) was taken on the front of the maytansinol peak (≅ 10 min) followed by a main fraction (Fr2, 3000 mL) and a small fraction of the tail (Fr3, 1400 mL). Fr1 had a purity of 90.8%, Fr2 of 99.4% and Fr 3 of 99.7%. The second and third fractions from the three runs were combined and the solvent removed using a rotary evaporator to give a residue. This residue was redissolved in 200 ml of ethyl acetate and the solvent removed again. The flask was pumped for 2 hours under high vacuum giving 9.3 g of purified maytansinol.

In another run under identical conditions, impure maytansinol (3.9 g) was dissolved in 89 ml of the mobile phase giving a concentration of ≅ 44 mg/ml. The analytical injection had a retention time of ≅ 14 minutes. The entire sample was purified in one injection giving 3.1 g of maytansinol at a purity of 99.3% by area. Differences in retention time can be attributed to the loading of the column, batch to batch variability of the stationary phase, variability of the mobile phase and temperature.

### Example 2 (Reference)

### Purification of Maytansinol by HPLC

Chromatography was carried out using a Hewlett Packard 1100 analytical HPLC system with solvent degasser, quaternary pump, autosampler, column oven and diode array detector. The system was controlled by Chemstation software. Crude maytansinol was prepared as described above and dissolved in the mobile phase at a concentration of 2 g/L. All experiments were carried out at 25°C. Typical injection volumes were 1 and 100 ul. Detection was carried out at a wavelength of 280 nm. HPLC-grade solvents were purchased from Mallinckrodt-Baker.

A 4.6 mm inside diameter x 250 mm stainless steel column was packed with a LICHROSPHER® Si 60, 10 µm silica stationary phase (E. Merck, Darmstadt, Germany). A mobile phase of 30% dichloromethane: 55% ethyl acetate: 15% 2-propanol was used at a flowrate of 2 mL/min.

200 ug of impure maytansinol prepared by lithium trimethoxyaluminum hydride reduction of ansamitocin P-3 was dissolved in the mobile phase as described above. Maytansinol was separated from all other peaks and eluted at 5 min with tailing to 8.5 min.

## Claims

1. A process for preparing maytansinol from a mixture comprising unreduced and over-reduced maytansinoids comprising separating the maytansinol by preparative HPLC on an angular particle porous amorphous silica gel stationary phase having a median pore diameter of 50-70 Å, a pore volume of 0.8-1.2 mL/g, a surface area of 500-600 m²/g, a packed density of 0.5 g/mL, <10% loss on drying, a 5% aqueous slurry pH of 4.0-5.5, sodium content of <60 ppm, aluminum content of <100 ppm, iron content of <80 ppm, calcium content of <80 ppm, sulfate content of <25 ppm and chloride content of <25 ppm eluted with a mobile phase of 50% dichloromethane : 39.3% ethyl acetate: 10.7% 2-propanol.

2. The process of claim 1 wherein the stationary phase has a particle size of 5, 10, 20, 40, 80 or 150 µm.

3. The process of claim 1 wherein the stationary phase is Silicycle IMPAQ®.

## Patentansprüche

1. Verfahren zur Herstellung von Maytansinol aus einer Mischung, die unreduzierte und überreduzierte Maytansinoide umfaßt, umfassend das Abtrennen des Maytansinols durch präparative HPLC an einer stationären Phase aus porösem amorphem Kieselgel mit kantigen Teilchen mit einem Medianwert des Porendurchmessers von 50-70 Å, einem Porenvolumen von 0,8-1,2 ml/g, einer Oberfläche von 500-600 m²/g, einer gepackten Dichte von 0,5 g/ml, <10 % Trocknungsverlust, einem pH einer 5 %igen wäßrigen Aufschlämmung von 4,0-5,5, einem Natriumgehalt von <60 ppm, einem Aluminiumgehalt von <100 ppm, einem Eisengehalt von <80 ppm, einem Calciumgehalt von <80 ppm, einem Sulfatgehalt von <25 ppm und einem Chloridgehalt von <25 ppm, eluiert mit einer mobilen Phase aus 50 % Dichlormethan : 39,3 % Ethylacetat : 10,7 % 2-Propanol.

2. Verfahren gemäß Anspruch 1, worin die stationäre Phase eine Teilchengröße von 5, 10, 20, 40, 80 oder 150 µm hat.

3. Verfahren gemäß Anspruch 1, worin die stationäre Phase Silicycle IMPAQ® ist.

## Revendications

1. Procédé de préparation du maytansinol à partir d'un mélange contenant des maytansinoïdes non réduits et sur-réduits qui comprend la séparation du maytansinol par HPLC préparative sur une phase stationnaire de gel de silice amorphe poreux à particules angulaires ayant un diamètre de pore médian de 50 à 70 Å, un volume de pores de 0,8 à 1,2 ml/g, une aire spécifique de 500 à 600 m²/g, une densité après garnissage de 0,5 g/ml, une perte au séchage inférieure à 10%, un pH d'une bouillie aqueuse à 5% de 4,0 à 5,5, une teneur en sodium inférieure à 60 ppm, une teneur en aluminium inférieure à 100 ppm, une teneur en fer inférieure à 80 ppm, une teneur en calcium inférieure à 80 ppm, une teneur en sulfate inférieure à 25 ppm et une teneur en chlorure inférieure à 25 ppm, éluée avec une phase mobile comprenant 50% de dichlorométhane : 39,3% d'acétate d'éthyle :10,7% de 2-propanol.

2. Procédé selon la revendication 1, dans lequel la phase stationnaire a une dimension particulaire de 5, 10, 20, 40, 80 ou 150 µm.

3. Procédé selon la revendication 1, dans lequel la phase stationnaire est du SiliCycle IMPAQ®.
